# FASCICULE DE BREVET EUROPEEN

(11) **EP 2 996 632 B1**
(45) Date de publication et mention de la délivrance du brevet: **10.03.2021**
(21) Numéro de dépôt: 13801608.4
(22) Date de dépôt: 14.11.2013
(51) Int. Cl.: A61F 2/24

(54) **PROTHESE ANNULAIRE POUR LA THERAPIE DE LA VALVE MITRALE**
RINGPROTHESE ZUR MITRALKLAPPENTHERAPIE
ANNULAR PROSTHESIS FOR MITRAL VALVE THERAPY

(30) Priorité: 16.11.2012 FR 1203069
(43) Date de publication de la demande: 23.03.2016
(73) Titulaire: Oder, Engin, 31170 Tournefeuille (FR); Zeybek, Rahmi, Istanbul (TR)
(72) Inventeur: Oder, Engin, 31170 Tournefeuille (FR); Zeybek, Rahmi, Istanbul (TR)
(86) Numéro de dépôt international: PCT/FR2013/000292
(87) Numéro de publication internationale: WO 2014/076379

(56) Documents cités:
- WO-A1-2009/139776
- WO-A2-2009/090564
- US-A- 4 917 698
- US-A1- 2005 131 533
- US-A1- 2005 256 569
- US-A1- 2006 217 803
- US-A1- 2008 154 359

## Description

La présente invention est une prothèse annulaire utilisée pour le traitement par annuloplastie des cas d'insuffisance de fermeture de la valve mitrale.

### 1 - Domaine, But de l'invention

L'invention a pour but la thérapie de l'insuffisance de la valve mitrale par annuloplastie.

### 2 - Description du Domaine Médical :

La valve mitrale est l'une des quatre valves qui séparent les quatre cavités du cœur. Elle se situe entre l'oreillette (atrium) gauche et le ventricule gauche.

La valve mitrale se ferme en phase systole et s'ouvre en phase diastole pour que la circulation du sang se fasse depuis l'oreillette gauche vers le ventricule gauche.

La valve mitrale est formée de deux membranes antérieure et postérieure fixées au cœur par l'intermédiaire de l'anneau mitral. Les membranes sont retenues par des cordages attachés au cœur dans le ventricule gauche par des piliers.

### 3 - Pathologie de l'Insuffisance Mitrale :

L'insuffisance mitrale apparait quand il y a altération de la valve mitrale.

Parfois cette insuffisance peut être due à un rétrécissement de l'anneau mitral.

Ce cas nécessite en général, le remplacement de la valve par une prothèse mécanique ou biologique.

Le plus souvent il s'agit de cas de fermeture insuffisante de la valve mitrale.

Le professeur Alain Carpentier a démontré que cette insuffisance à la fermeture de la valve mitrale est due à l'élargissement du segment postérieur de l'annulus.

Le cas d'insuffisance mitrale qui apparaît quand la longueur du segment antérieur reste constante, alors que le segment postérieur augmente. L'anneau mitral prend alors la forme d'un cercle aplati.

Cette déformation empêche la valve de se fermer efficacement. Les deux membranes de devant et de derrière de la valve qui ferment la surface de la valve n'arrivent plus à une coaptation complète pour une fermeture efficace.

Ceci dégrade l'efficacité de la fermeture de la valve et produit un reflux sanguin anormal dans l'oreillette gauche ce qui provoque l'insuffisance mitrale. Dans la plupart de ces cas, la réparation de la valve mitrale s'impose.

### 4 - Le traitement par annuloplastie de l'insuffisance de la valve mitrale

L'annuloplastie est une thérapie par intervention chirurgicale de remodelage des contours de la valve mitrale, associée ou non, au remodelage des membranes de la valve.

L'annuloplastie est une intervention conservatrice qui consiste à reconstituer la partie de la membrane de la valve déficiente, et à implanter un anneau afin d'éviter la dilatation annulaire de la valve. L'annuloplastie fixe le calibre de l'anneau mitral élargi au fil du temps au moyen d'une prothèse.

Avantageusement, une fois implantée, la prothèse de par sa construction fixe le calibre de la valve en contraignant le contour à garder son calibre originel.

Une prothèse articulée dont la projection sur un plan présente la forme de la lettre 'D' est connue du document WO-A-2009090564.

### 5 - Solutions actuelles et leurs inconvénients :

L'état de l'art actuel du traitement par annuloplastie de l'insuffisance mitrale se repose sur plusieurs techniques existant pour la plastie mitrale ou annuloplastie.

Plusieurs types de prothèses annulaires complètes ou partielles de différentes formes ont été inventés utilisant différentes technologies de remodelage des segments antérieur et postérieur de l'anneau mitral de l'annulus,

L'objectif des prothèses précédentes à notre invention objet de ce brevet, est le maintien statique du périmètre de l'anneau mitral.

Les techniques actuelles des prothèses antérieures à l'invention de notre prothèse objet de ce brevet sont classées en trois générations illustrées par les figures 1, 2, 3 annexées.
La figure 1 présente l'anneau mitral de Remodelage Rigide
La figure 2 présente l'anneau mitral de Remodelage Souple
La figure 3 présente l'anneau mitral de Remodelage Rigide en forme imagée de Selle de Cheval,

La plus ancienne de ces prothèses a été décrite par le professeur Alain Carpentier (1970).

Les prothèses de thérapie annulaire antécédentes à notre prothèse innovante objet de ce brevet, ont des inconvénients qui nuisent à leur efficacité thérapeutique de traitement chirurgical de l'insuffisance de la valve mitrale.

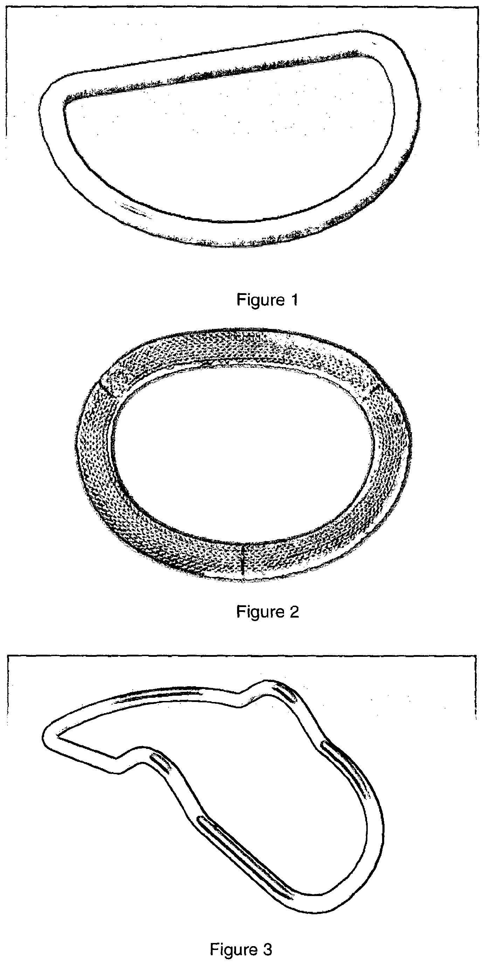

Les trois générations de l'état de l'art actuel des prothèses d'annulo-thérapie antérieures à l'invention de notre prothèse objet de ce brevet, ainsi que les inconvénients de chacune de ces générations sont :
1ère génération : Anneau de Remodelage rigide utilisé par le Dr. Carpentier. Présenté sur la figure 1, cette prothèse de remodelage (remodelling) de la première génération est un anneau (ring) rigide ayant la forme d'un haricot plat. Elle donne sa forme à l'annulus une fois cousue autour de la périphérie de la valve. Cet anneau contracte le segment postérieur de l'anneau et en même temps raccourci à sa valeur initiale la longueur du segment antérieur. C'est-à-dire fait un remodelage fixant le périmètre de l'annulus.
   Inconvénients :
   - Cette prothèse n'est pas satisfaisante car sa forme en anneau rigide et plat proche de l'état systolique de l'état mitral, lui permet pas une ouverture efficace de la valve en phase diastolique.
   - Ceci empêche une coaptation efficace des membranes de la valve qui nuit aussi à l'efficacité de la fermeture de la valve.
2ème génération : Anneau de Remodelage Flexible a été utilisé par Dr. Duran. Présentée sur la figure 2, cette prothèse de deuxième génération est un anneau souple, non extensible dans sa périphérie. La longueur fixe de la périphérie de l'anneau permet un remodelage fixant le calibre de la valve d'une façon souple. Il rétrécit le périmètre de l'anneau mitral de l'annulus sans contrainte de forme. Dr. Cosgroove a ajouté à ce groupe un anneau partiel qui rétréci l'annulus postérieur.
   Inconvénients :
   - Cette technologie de remodelage d'annulus s'est révélée moins efficace que la technologie de l'anneau rigide dans le traitement de l'insuffisance de la valve mitrale à cause de sa souplesse qui nuit à la coaptation et à l'ouverture efficace de la valve.
3ème génération : Anneau de Remodelage Rigide en forme imagée de Selle de cheval en trois dimensions. Présenté sur la figure 3, cette prothèse rigide de troisième génération a la forme imagée d'une selle de cheval conçue en trois dimensions correspondant à la forme systolique de l'anneau mitral en phase systolique de l'annulus. Cette forme est basée sur le résultat des études de la valve de mitral qui ont montré que la forme de l'annulus n'était pas plate mais avait la forme ressemblant à une selle de cheval (saddle shape).Il rétréci le périmètre de l'anneau et lui donne la forme d'une selle de cheval en phase systolique. Cette prothèse est citée dans la littérature en tant que "physio ring", qui est aussi le nom du fabriquant du même nom.
   Inconvénients :
   - Cette prothèse en anneau rigide a le désavantage de rester dans sa forme systolique sans pouvoir passer à la forme diastolique de l'annulus. Ce qui perturbe le flux sanguin à travers la valve mitrale par une ouverture inefficace en phase diastolique.

### 6 - La Solution Apportée par l'Invention objet de ce brevet :

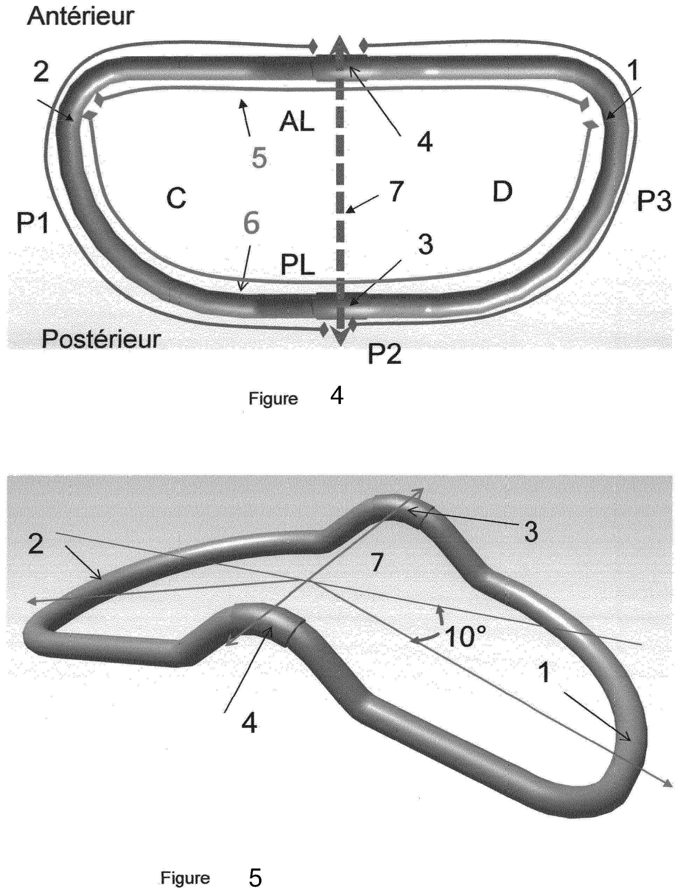

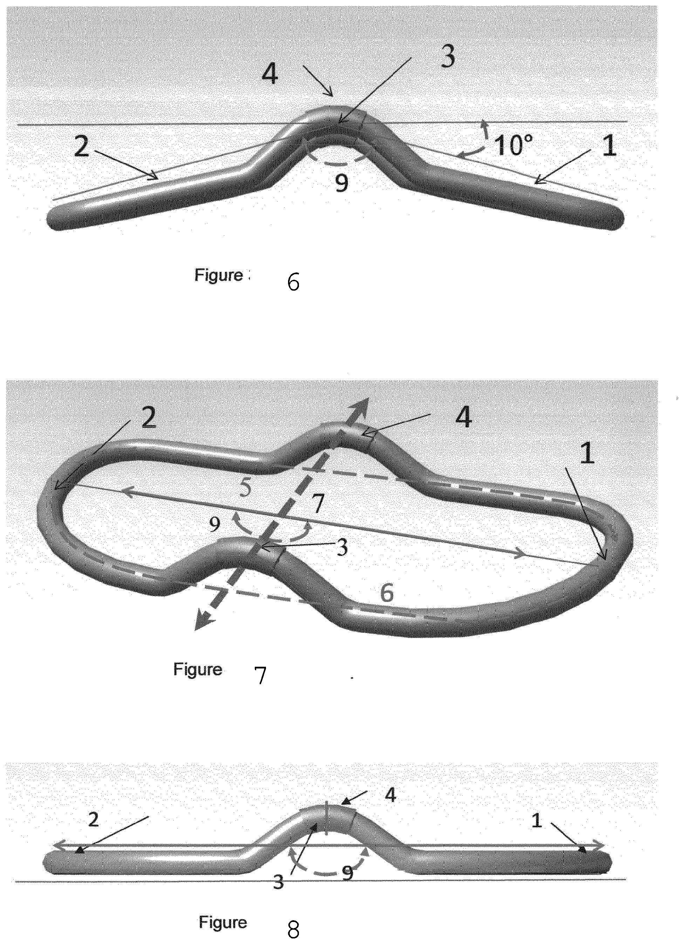

L'invention, objet de ce brevet, est une prothèse destinée au remodelage dynamique en quatre dimensions, dans l'espace et dans le temps, de l'anneau de la valve mitrale. Elle épouse dynamiquement les formes de l'anneau mitral de la valve mitrale depuis sa forme systolique (figures5,6), jusqu'à sa forme diastolique(figures7,8), suivant les phases du mouvement du cœur.

Avantageusement la prothèse innovante d'annulo-plastie, objet de ce brevet, répond efficacement aux problèmes d'insuffisance de la valve mitrale liés à la déformation pathologique de l'anneau mitral.

Avantageusement, la prothèse, objet de ce brevet, de par sa constitution, contraint l'anneau mitral à prendre les formes naturelles tridimensionnelles originelles à la valve mitrale pour assurer le flux efficace du sang.

Avantageusement, en phase systolique la prothèse, de par sa constitution innovante, donne la forme imagée d'une selle de cheval (figures5, 6, 7, 8) à l'anneau mitral.

Avantageusement la coaptation des deux membranes de devant et de derrière de la valve est alors complète pour une fermeture efficace contre le reflux du sang.

Avantageusement, en phase diastolique, la prothèse, objet de ce brevet, de par sa constitution innovante, contraint l'anneau mitral à prendre la forme imagée d'une selle de cheval aplatie en forme de la lettre 'D' (figure 7,8). Cecei augmente la section de la valve en phase diastolique.

Avantageusement une ouverture complète des deux membranes de devant et de derrière est obtenu par la forme de la selle de cheval aplatie de l'anneau facilitant ainsi le flux sanguin à travers la valve mitrale.

### 7 - En quoi l'Invention résout-elle le problème :

Avantageusement, la prothèse d'annulo-plastie innovante, objet de ce brevet, ne présente pas les inconvénients des prothèses antécédentes.

Avantageusement, pour résoudre le problème d'insuffisance de la valve mitrale, la structure innovante de la prothèse est en accord avec les conclusions des dernières études en la matière.

Ces études de la cinématique de la valve mitrale ont montré que l'annulus a la forme imagée d'une selle de cheval (saddle shape) qui se déploie et se rétracte dans un mouvement de battement autour des deux sommets à la manière imagée d'un papillon, entre les états systole et diastole de la forme rétrécie à la forme aplatie de la selle.

Avantageusement, de par sa constitution physique innovante, notre prothèse, objet de ce brevet, impose à l'anneau de la valve mitrale reconstituée les mouvements en accord avec les résultats des dernières études de la cinématique de la valve mitrale.

Avantageusement, la prothèse, objet de ce brevet, de par sa constitution physique, reconstitue la forme de l'anneau mitral articulé dans l'axe médiane des annulus postérieur et antérieur par deux zones charnières.

Avantageusement, cette configuration dynamique de la prothèse, supprime les inconvénients des prothèses précédentes et résout efficacement l'insuffisance de la valve mitrale abimée :
- régurgitation (reflux) du sang par une fermeture dégradée de la valve dans son état systole et
- l'ouverture inefficace de la valve pour un flux sanguin dans son état diastole des anciennes prothèses.

En effet, avantageusement, la prothèse de l'anneau mitral, l'objet de ce brevet, donne les formes naturelles à la valve mitrale. De la forme imagée d'une selle de cheval, la prothèse est formée de deux segments rigides constituant le contour gauche et le contour droit de la selle de cheval réunis aux deux sommets par des charnières. La prothèse s'élargit en s'aplatissant à l'ouverture de la valve pour assurer une section plus grande au flux sanguin. Elle se rétracte à la fermeture de la valve.

Avantageusement, l'efficacité de l'innovation de notre brevet se trouve dans le fait que les charnières qui sont placées aux sommets de la forme imagée d'une selle de cheval qui impose au contour de la valve mitrale dans un **mouvement de rotation dans l'axe annulus antérieur** - **postérieur**, les formes Systolique et Diastolique au originelle à la valve.

Ainsi, avantageusement notre dispositif objet de ce brevet, impose à la valve mitrale :
- **dans son état systolique** une forme imagée d'une selle de cheval en se rétractant autour de ses deux lobes gauche et droite dans l'axe annulus postérieur - antérieur, ce qui constitue une forme optimale pour la fermeture de la valve,
- **dans son état diastolique**, une forme vue de dessus, aplatie proche de la lettre 'D' qui améliore le flux sanguin en augmentant la section effective de la valve.
- **grâce à ses charnières** fabriquées en matières **flexibles** ou **articulées à pivot**, ont par constitution des débattements limités par des butées de fin de course.

### 8 - Meilleur mode de réalisation de l'invention

La mode de réalisation de la prothèse de l'anneau mitral d'annulo-plastie selon l'invention est constituée d'un anneau formé de deux demi-segments rigides (1)(2), et de deux articulations (3)(4) reliant les extrémités respectives des demi-segments pour former un anneau en forme imagée de selle de cheval.

La prothèse selon l'invention est un anneau formé de deux segments (1)(2) articulés aux sommets de la forme imagée d'un selle de cheval, qui se rétractent (figures 5, 6) et se déploient (figures 7,8) à la manière imagée de battement d'aile d'un papillon. passant ainsi de la position systole à la position diastole de l'anneau mitral, dans un mouvement de battement autour des zones charnières (3)(4) autour d'un axe (7) passant par les deux sommets.

Les figures annexées 4, 5, 6, 7,8 présentent les positions de la prothèse dans :
- La figure 4 : l'anneau mitral vue de dessus
- La figure 5 : la vue perspective de l'anneau mitral dans son état systole
- La figure 6 : la vue de face de l'anneau mitral dans son état systole
- La figure 7 : la vue perspective de l'anneau mitral dans son état diastole
- La figure 8 : la vue de face de l'anneau mitral dans son état diastole

La figure annexée 4 présente la forme imagée de la lettre D de la prothèse vue de dessus de l'anneau mitral. Le côté long (5) courbé en forme d'arc de la lettre 'D', constitue l'annulus postérieur et le côté court (6) constitue l'annulus antérieur.

L'anneau mitral passe en phase systole (figure 5 et 6) en se rétractant autour d'un axe (7) passant par les milieux de l'annulus postérieur (5) et celui de l'annulus antérieur (6) pour prendre la forme imagée de la selle de cheval. La valve est alors fermée au flux sanguin par une coaptation efficace des membranes et un rétrécissement de la surface projetée de la section de la valve.

L'anneau de la valve mitrale dans son état diastole (figures 7 et 8) s'aplatie dans un mouvement où la longueur de l'annulus antérieur (6) reste fixe, alors que l'annulus postérieur (5) s'allonge et se rapproche d'une forme arrondie avec la surface de la section projetée élargie. La valve s'ouvre alors au flux sanguin. Avantageusement la prothèse, objet de ce brevet, est constituée :
- **de deux segments rigides** gauche et droite (1)(2) articulés entre eux à leurs extrémités respectifs par deux charnières(3)(4). Le segment gauche (1) formant la partie gauche de l'anneau mitral en partant du milieu de l'annulus postérieur, jusqu'au milieu de l'annulus antérieur, le segment droite (2) formant la partie droite de l'anneau mitral en partant du milieu de l'annulus postérieur, jusqu'au milieu de l'annulus antérieur,
- **de deux articulations** charnières (3)(4) à débattements limités par des butées réalisées en matière biocompatible réunissent les deux segments (1)(2) l'une au milieu de l'annulus postérieur et l'autre au milieu de l'annulus antérieur.

La prothèse, objet de ce brevet, est réalisée préférentiellement en deux versions selon la nature des charnières (3)(4) :
- La prothèse à articulations par zones flexibles,
- La prothèse à articulations par charnières à mécanisme à pivot.

Selon l'invention, les deux versions de la prothèse sont articulées par leurs articulations situées au milieu de l'annulus antérieur et de l'annulus postérieur (3)(4) constituant l'anneau mitral.

Dans la version flexible par zone, la prothèse s'articule par des zones faites en matières biocompatibles (1)(2) qui jouent le rôle d'articulation dans la continuité dimensionnelle de l'anneau mitral.

Dans sa version articulée par des charnières mécaniques en matière biocompatible, les deux segments (1)(2) sont reliés par des pivots à butées mécaniques (3)(4) pour contraindre les mouvements de l'anneau mitral de la valve mitrale au mouvements imagée d'une selle de cheval.

### 9 - Avantages de l'invention :

Avantageusement, notre prothèse selon l'invention, est un anneau mitral qui donne à la valve mitrale reconstituée, les formes conformes aux dernières constatations cliniques de la valve mitrale saine, présentées d'une façon usuelle dans la littérature spécialisée.

Avantageusement aux prothèses antécédentes, la prothèse, objet de ce brevet, impose dynamiquement à la valve mitrale les formes systole (figures 5,6) et diastole (figures 7,8) grâce aux articulations (3)(4) des segments (1)(2) de la selle de cheval.

La prothèse, objet de l'invention, remplace avantageusement les prothèses antécédentes en imposant à la valve mitrale des mouvements naturels et sains grâce à l'anneau mitral articulé, qui contraint la valve reconstituée aux mouvements en trois dimensions, systole à diastole naturels.

Les figures 5,6 et 7,8 annexées, illustrent les mouvements de la prothèse, objet de ce brevet, qui montre le mouvement de l'anneau mitral pour passer au plus près de la forme systole à la forme diastole en suivant les phases du battement du cœur.

Avantageusement, les articulations à butés fixent l'amplitude des battements de chacun des deux segments (1)(2) entre 0 à 10 degrés d'angle autour de l'axe (7), de part et d'autre de la verticale par rapport à l'horizontal de l'anneau mitral.

Avantageusement aux prothèses antécédentes, les articulations permettent à la prothèse de se mouvoir entre les deux formes extrêmes systolique et diastolique tout en fixant le calibre de l'anneau mitral à sa valeur saine par remodelage de la périphérie de la valve mitrale à sa forme originelle.

Avantageusement aux prothèses antécédentes, l'anneau mitral de la prothèse objet de ce brevet, se rétracte grâce à ses articulations à butés, pour passer à l'état systolique rétréci (figures 5,6).

Avantageusement aux prothèses antécédentes, la prothèse, objet de ce brevet, remodèle la valve mitrale dans la forme rétrécie de la selle de cheval.

Avantageusement aux prothèses antécédentes, la prothèse, objet de ce brevet, dans sa forme rétrécie, permet la coaptation efficace des membranes de la valve mitrale qui élimine tout reflux sanguin dans la valve mitrale.

Avantageusement aux prothèses antécédentes, en phase diastolique, l'anneau mitral de la prothèse objet de ce brevet, s'ouvre jusqu'à prendre la forme aplatie (figures annexées 7,8) qui offre une section effective élargie au flux sanguin.

Avantageusement aux prothèses antécédentes, en phase diastolique, l'anneau mitral de la prothèse objet de ce brevet, donne la forme diastolique originelle à la valve mitrale.

Avantageusement, la prothèse objet de ce brevet, en phase diastolique, offre une meilleure au flux sanguin par rapport aux solutions antécédentes.

Avantageusement, la prothèse annulaire, objet de ce brevet, est dimensionnée selon le contour originel de la valve mitrale du receveur.

## Revendications

1. Prothèse annulaire pour la thérapie de la valve mitrale par annuloplastie, destinée à être implanté sur le contour de la valve mitrale pour la thérapie par remodelage du calibre de l'anneau de la valve mitrale par annuloplastie, s'agissant d'un anneau à la forme originale de la valve mitrale pour guider ses mouvements de l'état systolique jusqu'à l'état diastolique, la prothèse étant constituée :
- d'un **anneau fermé** formé de deux segments (demi-anneaux) rigides (1), (2) constituant deux lobes, l'annulus antérieur (5) et l'annulus postérieur (6) de la valve mitrale, articulés entre eux par des zones charnières à butées (3), (4), pour les battements des lobes limités par les butées,
- les **deux charnières à butées** (3), (4) s'articulent entre 0° et 10° fixé par les butées, fixant l'axe de rotation (7) passant par les milieux de l'annulus postérieur (6) et de l'annulus antérieur (5),
- les **deux segments rigides** (1), (2), partant du milieu du segment d'annulus postérieur (6), jusqu'au milieu de l'annulus antérieur (5), et se déployant jusqu'à la butée supérieure des charnières d'un angle de 0° pour l'état **diastolique** (valve ouverte - forme de D aplati) et se rétractant jusqu'à la butée inférieure d'un angle de 10° pour prendre l'état **systolique** (valve fermée).

2. Dispositif selon la revendication 1, **caractérisé en ce que** les articulations charnières entre les segments, sont réalisés par des **charnières à mécanisme à pivot et des butées mécaniques**.

3. Dispositif selon la revendication 1, **caractérisé en ce que** les articulations charnières (3), (4), reliant les segments, sont réalisés par des **zones flexibles en matière biocompatible** qui contraignent les battements des lobes entre 0° et 10° et réalisent ainsi la fonction de butées.

## Patentansprüche

1. Vorrichtung in Form einer Ringprothese, die mittels einer Reparaturring-OP um die äußeren Konturen der Herzklappe eingesetzt wird,**dadurch gekennzeichnet**:
- ein **geschlossener Ring**, mit der die zwei festen Abschnitte (1) (Halbring) (2) gebildet werden, die die zwei Lappen (5), (6) der Herzklappe (2) und die axialen Drosselungsbereiche (3), (4) bilden, die eine Begrenzung beim Schlagen der Lappen bewirken,
- Zwei Begrenzungsgelenke (3), (4), die eine Öffnung in einem Winkel von 0 bis 10 Grad ermöglichen, die durch die Begrenzungskomponenten bestimmt werden und die Drehachse (7) bestimmt wird, die zwischen dem hinteren Ring (6) und dem vorderen Ring (5) verläuft;
- **Zwei feste Abschnitte**, die sich von der Mitte des hinteren Ringabschnitts (6) bis zur Mitte des vorderen Ringabschnitts erstrecken und sich im Diastolenzustand (offenes Ventil - flache D-Form) in einem Winkel von 0 Grad zum oberen Begrenzer der Gelenke bewegen und im Systolenzustand (geschlossenes Ventil) in einem Winkel von 10 Grad zum unteren Begrenzer zurückziehen, sind Bestandteile von (1), (2),

2. Vorrichtung nach Anspruch 1, **dadurch gekennzeichnet, dass** die **Gelenkverbindungen zwischen den Abschnitten im Herzen mit Gelenken mit einem Axialmechanismus und mechanischen Begrenzern** hergestellt sind,

3. Vorrichtung nach Anspruch 1, **dadurch gekennzeichnet, dass** die die Abschnitte verbindenden Verbindungen (3), (4) aus flexiblen Bereichen aus biokompatiblem Material bestehen, die die Pulsation der Lappen zwischen 0 und 10 Grad einschränken und deren Begrenzungsfunktion bereitstellen.

## Claims

1. Device forming an annular prosthesis intended to be attached (around) to the contour of the mitral valve by annuloplasty therapy, **characterized in that** it contains:
- a **closed ring** consisting of two rigid (1) segments (half-rings), (2) constituting (of) the two lobes (5), (6) of the mitral valve, articulated between them by pivotal stop zones (3), (4), for the flapping of the lobes limited by the stops,
- the **two hinges with stops** (3), (4) are articulated between 0 and 10 degrees as determined by the stops, fixing the axis of rotation (7) passing through the middles of the posterior annulus (6) and the anterior annulus (5),
- the **two rigid segments** (1), (2), from the middle of the posterior annulus segment (6), to the middle of the anterior y (5), and moving to the upper stop of the hinges at a 0-degree angle for **diastolic** state (open valve - flattened D shape) and retracting to the bottom stop at an angle of 10 degrees to take the systolic state (closed valve).

2. Device according to claim 1, **characterized in** the heart that the hinged joints between the segments, are made by **hinges with pivot mechanism and mechanical stops**.

3. Device according to claim 1, **characterized in that** the joints (3), (4), connecting the segments, are made by **flexible areas of biocompatible material** that constrain the flapping of the lobes between 0 and 10 degrees and thus achieve the function of stops.
